Europäisches Patentamt

European Patent Office

Office européen des brevets

(11) Publication number: **0 304 947**
**A2**

# EUROPEAN PATENT APPLICATION

(21) Application number: **88113988.5**

(22) Date of filing: **26.08.88**

(51) Int. Cl.⁴: **C12M 1/40**

(30) Priority: **27.08.87 JP 213783/87**

(43) Date of publication of application:
**01.03.89 Bulletin 89/09**

(84) Designated Contracting States:
**BE DE FR GB**

(71) Applicant: **Tosoh Corporation**
**4560, Oaza Tonda**
**Shinnanyo-shi Yamaguchi-ken(JP)**

(72) Inventor: **Kagayama, Toshi**
**35-21, Sakuradai Midori-ku**
**Yokohama-shi Kanagawa-ken(JP)**

(74) Representative: **Vossius & Partner**
**Siebertstrasse 4 P.O. Box 86 07 67**
**D-8000 München 86(DE)**

(54) **Biosensor.**

(57) A biosensor comprises at least a pair of flat plates (10, 13) placed opposingly to each other forming fine spacing therebetween, a physiologically active substance (14) fixed on a surface of one of the plates (13) facing the space, and a first transducer (11, 12) responsive to a chemical substance provided on a surface of the other plate (10) facing the space wherein the space is utilized for accommodating a sample solution

F I G.1(a)

## Biosensor

The present invention relates to biosensors which are produced by combining a physiologically active substance with a transducer which is responsive to certain chemical substances. More particularly, the invention relates, for example, to a biosensor which permits a particular substance present in a small amount of a biological sample to be readily determined without agitating the sample.

A variety of sensors are so far proposed. They are, for example, a sensor utilizing a glass electrode and an ion sensor which uses a field effect transistor as transducer.

These sensors are composed of a responsive transducer such as glass electrode or field effect transistor which is coated with a functional membrane, where the membrane is made of a high molecular or porous membrane on which surface is installed an antigen, antibody or an enzyme (hereinafter they are collectively called physiologically active substances). Change of the circumstance in which a particular substance increases or decreases as a result of an immunological reaction of above antigen and antibody or of an enzymatic reaction is detected electrically as a change of surface potential of the responsive part of the transducer. Thus, the output itself or a change of the output of the electrical signal enables the particular substance to be detected and determined.

Among the sensors composed as mentioned above, those which employ an glass electrode require an internal liquid for the electrode itself. Therefore problem is that an essential difficulty is encountered in producing sensors of a small size. This problem induces an additional difficulty that a sample room of a small size is not attainable for this sensor since a sample needs to be at least several cubic centimeters, when the sensor is used in either a batch or flow form.

On the other hand, when a sensor is produced from a transducer of a field effect transistor, that is the latter type of biosensors mentioned above, together with a functional membrane which covers the transducer and on which surface an enzyme or so is installed, the sample size can be characteristically reduced down to about 500 $\mu$l. However, an attempt to further decrease the sensor size by reducing the volume of sample solution for the estimation introduces another serious difficulty in the operation of covering the transducer uniformly and stably with an enzyme membrane. This procedure may very probably produce pinholes and cracks. Even if the problem concerning the pinholes and cracks comes to be solved, presence of the membrane itself interferes with the passage of the chemical substances to be estimated, and so much lowers the sensitivity.

Furthermore, when a biosensor of a conventional type is used and a sample solution is injected to the sensing portion in a dipping or dropping fashion, diffusion of the responsive material of the transducer may be influenced by the rocking and flowing motion of the liquid and it may cause to decrease the precision of estimation.

The present inventors made efforts to solve the problems concerning conventional biosensors as mentioned above. They overcame the problems by fixing on a plate a physiologically active substance which produces a transducer responsive material, installing on another plate a responsive part of the transducer to detect the responsive material and by placing the two plates opposingly facing to each other with a sample solution between the two plates. thus, they succeeded in composing a biosensor for a minute volume of sample solution as an object without any trouble and completed the present invention.

The biosensor of the present invention which is created to meet this end comprises having at least a pair of plates with flat surfaces which opposingly face to each other with a small spacing between them, fixing a physiologically active substance on the surface of one of the plates which faces to the space, installing a transducer responsive to certain chemical substances on the surface of the other plate, and making the space between the plates to accommodate a sample solution.

The small spacing between the two plates mentioned above can be maintained constant using appropriate spacers placed somewhere between the two plates.

With the biosensor of the present invention thus composed, the responsive part of the transducer can be disposed to allow direct contact with a sample solution where a substance to which the transducer is responsive is formed. In comparison with previous apparatus in which substances to be sensed have to penetrate a functional membrane, the estimation can be performed without influence of a membrane and therefore with a merit of improved precision.

Since the physiologically active substance can be fixed on a plate different from that on which the transducer is installed, a homogeneous layer of the physiologically active substance is formed more easily on the surface as compared with the case when an enzyme is fixed on a functional membrane and the functional membrane is fixed to a responsive part of a transducer, as it is the case with a previous apparatus.

When the sensor is composed as described above, the plate on which the physiologically active substance is fixed may be made from glass or high polymers of vinyl chloride and acrylate. The physiologically active substance to be used and fixed on the plate should be one the substrate of which is the object substance to be estimated and produces or consumes, as a result of a chemical reaction, a substance that the transducer is responsive to. Enzymes include, for example, glucose oxidase, choline oxidase, uricase and urease. On composing the apparatus, an enzyme particularly selected according to the object material of estimation should be fixed on the plate.

Fixing a physiologically active substance on a plate may be carried out, for example, by the occlusion method in which an enzyme such as glucose oxidase is fixed by being occluded in a matrix of a high molecular compound such as photo-cross linked polyvinyl alcohol, by the adsorption method in which a physiologically active substance is fixed on an organic membrane such as LB membrane, and by the covalent bonding method in which a physiologically active substance together with bovine serum albumin (BSA) in an aqueous solution crosslinkingly fixed with glutaric aldehyde.

The transducer of this invention which is installed on the other plate and is responsive to certain chemical substance is capable of detecting concentration, or change of concentration, of an object chemical species in the solution and transforming it into an electrical signal. An example is a thin film electrode of an electroconductive material such as gold, platinum, and graphite which is formed by the vacuum evaporation or screen printing method, for example, on a glass plate. The proton responsive ISFET is another example. The proton responsive ISFET is a modification of MOS type FET, in which the metal film on the insulation membrane at the gate is replaced by an ion responsive membrane and can be produced according to the process described in Japanese Laid-Open Patent Application No. Sho 57-76043.

The two plates prepared as described above are held in opposing positions to each other with a small spacing between them. Since a sample solution should be held in the space, it is advised the magnitude of the spacing is so determined that the solution can be held without dropping by the adhesive force of solution toward the plates. The value of the spacing depends on the sensitivity of measurement and the speed of response, as well as on the nature and the concentration of sample solution. Therefore the most appropriate spacing is determined considering the nature of the sample solution and the required precision of estimation. When a minute volume of solution is considered, a spacing ranging from 0.1 - 3 mm is often referred.

The spacing described above may be maintained at a constant value by any adequate, but not restricted, way such as adhering the two plates with spacers between them or fixing the two plates to a supporting holder and molding the whole.

The biosensor of this invention is ordinarily composed in such a structure that a plate with a physiologically active substance fixed on it and another plate with a transducer installed on it are supported by an adequate holder so that the relative position of the two plates are settled to each other. However, the plate on which a physiologically active substance is fixed may be made replaceable depending on the items of estimation. Otherwise, pairs of the two plates as mentioned above are disposed in parallel so that the sample solution can be analyzed and estimated on a plurality of items at the same time.

Into the space of the biosensor thus formed for accommodating a sample solution, the solution is placed in any convenient manner, such as by injection or by soaking the sensor into the solution.

In addition to the above mentioned configuration, the biosensor of the present invention is generally completed by inclusion of a comparison electrode with which to estimate the electrical potential of a sample solution, and an external measuring device which is connected to the transducer and the comparison electrode.

Brief Explanation of the Drawings:

Figs. 1(a), 1(b) and 1(c) are schematic diagrams with which to explain the structural outline of the glucose sensor of batch type composed, as an example, according to the present invention. Fig. 1-(a) is a side view of a glucose sensor, Fig. 1(b) is a cross section along line A - A in Fig. 1(a) and Fig. 1(c) is a cross section along line B - B in Fig. 1(a).

Fig. 2 shows the measuring circuit to be connected to the glucose sensor and Fig. 3 is a calibration line with which to estimate the glucose content when estimation is made with the glucose sensor appearing in Fig. 1.

Figs. 4(a), 4(b), 4(c) and 4(d) are schematic diagrams with which to explain the structural outline of the urea sensor of batch type composed, as an example, according to the present invention. Fig. 4(a) is a partial side view of the urea sensor, Fig. 4(b) is a cross section along line C - C in Fig. 4(a), Fig. 4(c) is a cross section along line D - D in Fig. 1(a) and Fig. 4(d) is a cross section along line E - E in Fig. 1(a).

Fig. 5 is an enlargement of the electrode and the vicinity of the ISFET responsive to protons.

Fig. 6 is a measuring circuit connected to the urea sensor and Fig. 7 is a calibration curve for the concentrations of urea when estimation is made with the urea sensor appearing in Fig. 4.

Figs. 8(a), 8(b) and 8(c) and Figs. 9(a), 9(b), 9(c) and 9(d) are applicable to other examples.

The present invention will be better understood from the following description of preferred embodiments with reference to the attached drawings.

Example 1

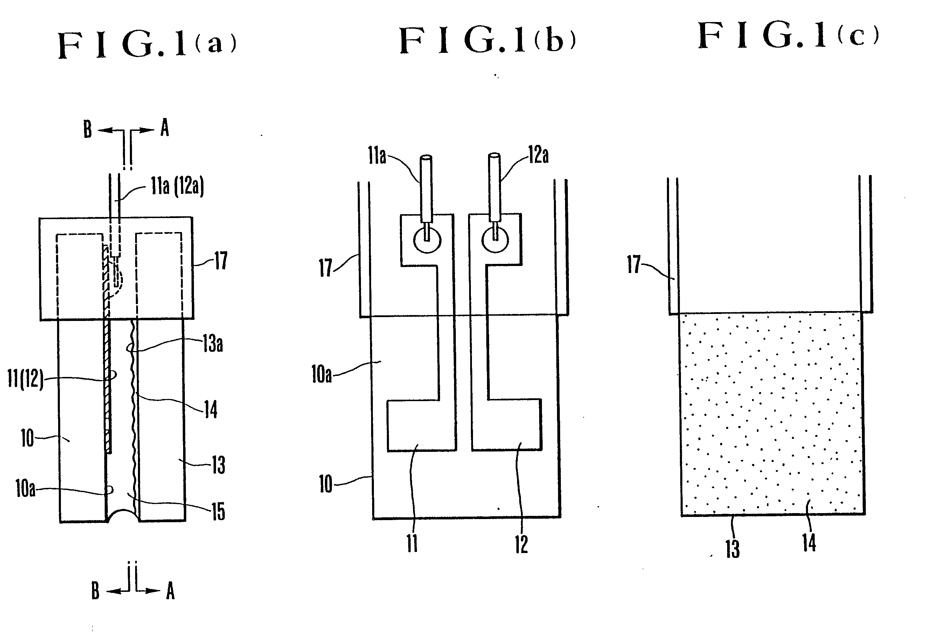

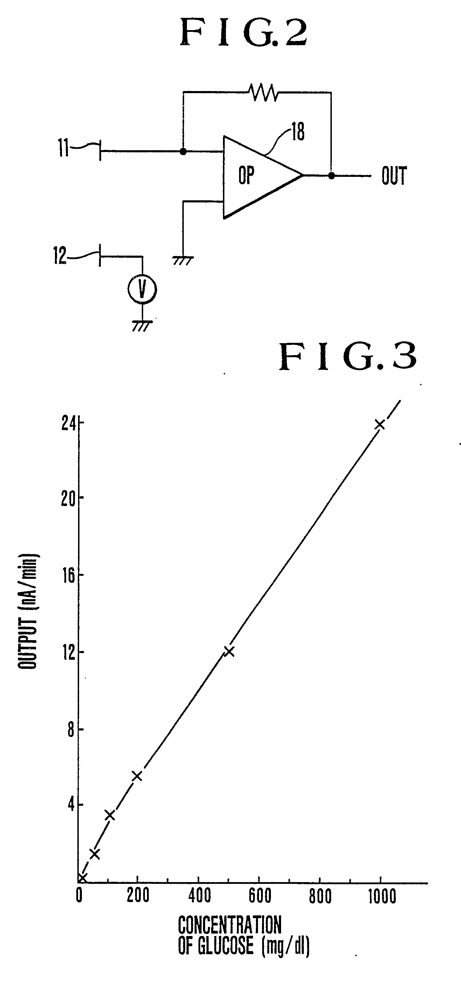

Figs. 1 to 3 are provided to explain the present invention when it is embodied in composing a glucose sensor of a batch type. Figs. 1(a), 1(b) and 1(c) show the structure of the sensor and Fig. 2 shows the measuring circuit which is connected to the biosensor.

In these figures, 10 is the first glass plate on which a transducer is installed and is supported by the holder 17 together with the second glass plate, which will be mentioned below, the two plates being settled opposite and in parallel to each other with a small spacing between them and the whole is molded all together. On the surface 10a of the first glass plate 10 which faces the second glass plate, an anode 11 and a cathode 12 are prepared as thin gold electrodes of the form illustrated in Fig. 1(b).

Each electrode mentioned above is connected to the measuring circuit in Fig. 2 via a lead wire 11a or 12a.

The object 13 is the second glass plate. On the surface of the plate 13a which faces the first glass plate are fixed laminate films 14 of physiologically active substance in the form of mixed LB films of stearyl trimethyl ammonium and arachic acid methyl which contain adsorbed glucose oxidase. The second glass plate 13 is fixed together with the first plate to the holder 17 by being molded all together.

The first plate 10 and the second plate 13 are arranged so that the electrode and the fixed enzyme face each other as illustrated in Fig. 1(a) and the inbetween spacing is settled to be 1.0 mm in this example. The space thus prepared forms a sample room 15 where a sample solution added can be held without being dropped.

Fig. 2 is the measuring circuit which is connected to the three electrodes mentioned above and is operated by the direct current voltage source V connected to the cathodic electrode 12. With aid of the circuitry containing an operational amplifier 18, concentration of a certain ion in the sample solution detected at the anodic electrode 11 in the form of an electric potential is transformed into an output signal of this measuring circuit.

Using the glucose sensor thus composed (with an area of 0.80 cm x 0.60 cm for each of the facing plates) and 50 $\mu$1 of a glucose solution injected in the sample room 15 and with a bias voltage of +700 mV applied between the anodic 11 and the cathodic electrodes, the amount of hydrogen peroxide produced on the decomposition of glucose by the action of glucose oxidase was estimated from the initial rate of formation of hydrogen peroxide by means of the amperometric method.

An example of the calibration line for the glucose sensor is shown in Fig. 3. The result makes us confirm that a sufficiently high precision of estimation could be obtained with as minute as 50 $\mu$1 of a solution.

Example 2

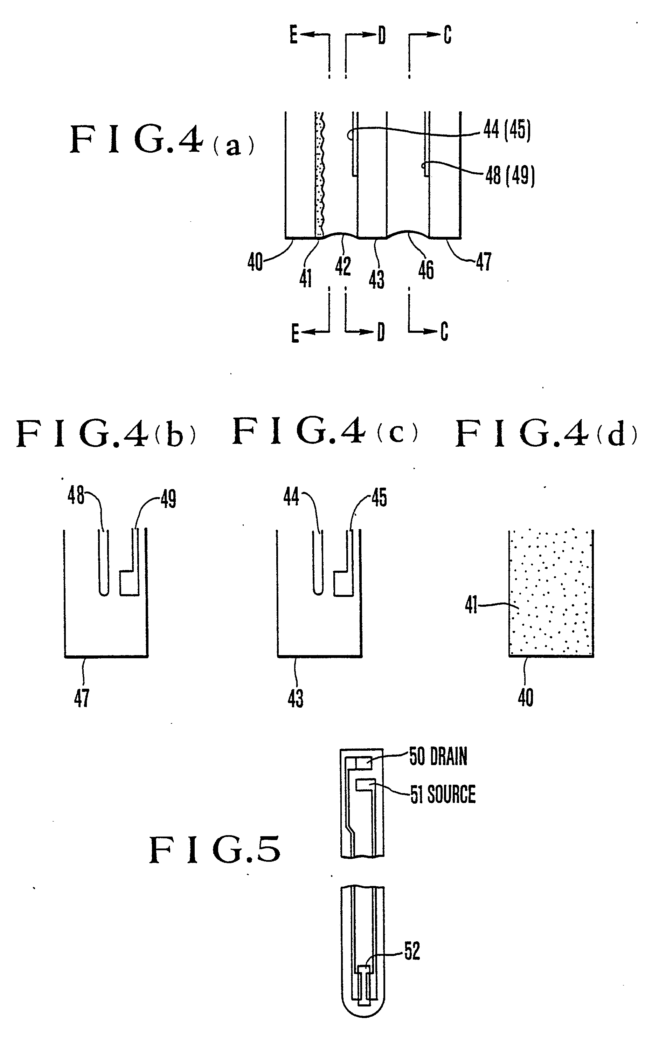

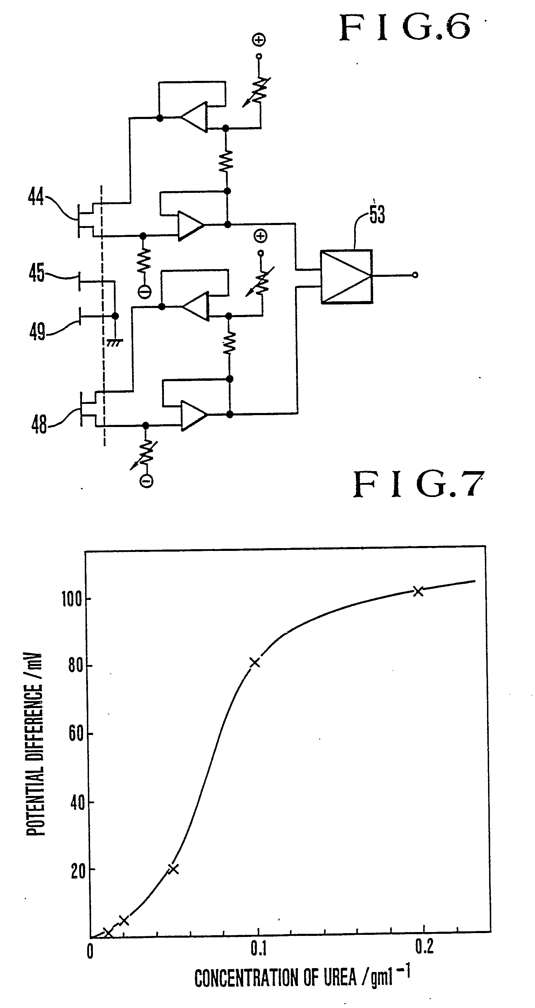

Figs. 4 to 7 explain the example in which the present invention is applied to compose a sensor for urea of the batch type. Figs. 4(a), 4(b), 4(c) and 4(d) show the conformational structure of the sensor and Fig. 6 shows a measuring circuit connected to the biosensor.

In these figures, 40 is the second plate holding on its surface the films 41 fixing a physiologically active substance which, in this case, is urease. 43 is the first plate on which surface are installed an ISFET 44 responsive to protons for measurement and a pseudo counter electrode 45. 47 is the third glass plate installed on its surface an ISFET 48 for comparison which is responsive to protons and a pseudo counter electrode 49. The three plates are arranged as is seen in Fig. 4(a), where the first plate 43 is placed with the electrodes on the surface faced toward the films 41 fixing the physiologically active substance on the second plate 40 and, on the rear side of the first plate 43, the ISFET 48 responsive to protons for comparison on the third plate 47 is placed with the ISFET facing the first plate 43 and the three plates are held with small spacings (each 1.0 mm in this example) in parallel to each other. The plates are supported and settled with a holder not shown in figures in the same way as in Example 1.

In the structure prepared with the three plates, the empty space produced between the second plate 40 and the first plate 43 is the sample room 42 for accommodating a sample solution for estimation, and the space between the first plate 43 and the third plate 47 forms the comparison sample room 46 for accommodating a comparison sample solution.

These electrodes are connected to a constant gate voltage circuit shown in Fig. 6. Variation of pH of a sample solution is estimated as difference in potential of the two ISFET's responsive to protons, one is for measurement 44 and the other is for comparison 48. Calculation to obtain the difference is performed with a subtractor 53 at the output of the measuring circuit. In the measuring circuit of this example, variation of the surface potential superimposes the gate voltage of ISFET and the drain current varies. In the actual operations, however, the drain current and the drain-source voltage are maintained constant, so that the variation of surface potential at the interface of the gate membrane and the sample solution which the ammonium ions generated when urease in a sample solution decomposes urea cause to elevate the pH of the sample solution could be detected from the change (or decrease): of the source-drain current.

Fig. 5 is an enlargement of the electrodes on the proton responsive ISFET's 44 and 48, which are formed, as are well known, from drain 50, source 51 and gate membrane 52 responsive to protons.

Using a sensor for urea which is composed as described above, each 30 $\mu$1 of a urea solution was injected into each of the sample rooms, 42 and 46, and in a certain length of time difference of the pH's in the two rooms was estimated. In this example, estimation was made with the drain current (Id) of the proton responsive ISFET being 100 $\mu$A and the drain-source voltage (Vd) being kept at 5V.

A calibration curve connected with the urea sensor is partly shown in Fig. 7. As confirmed from the figure, a sample solution of as minute a volume as 30 $\mu$1 is sufficient to obtain a result of high precision.

Further, the present invention is not restricted to the above examples, but can be achieved in form of different modifications.

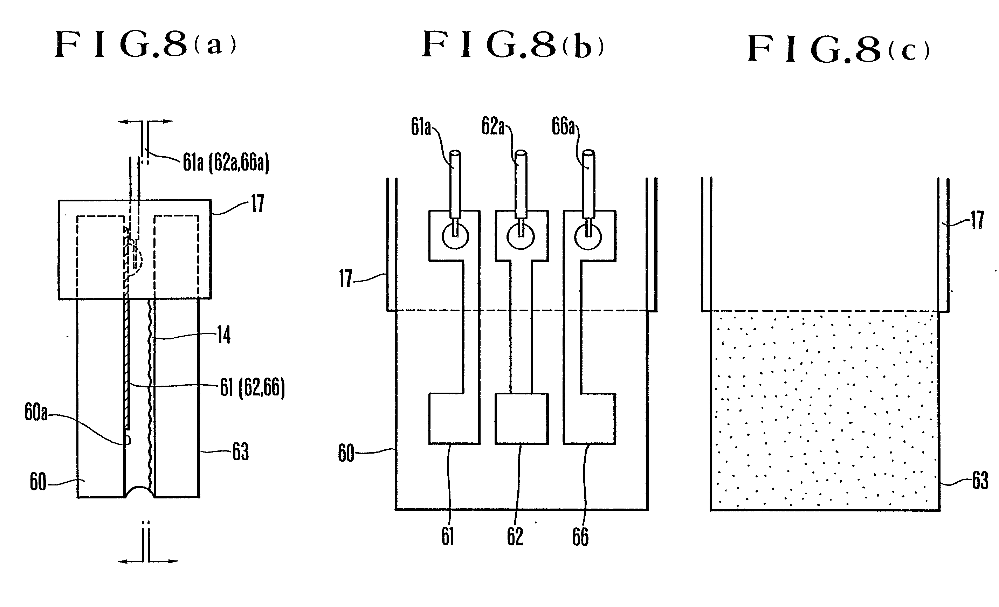

In the embodiment shown in Figs. 8(a), 8(b) and 8(c) where the first plate 60 and the second plate 63 are held in opposite and parallel to each other, for example, a physiologically active substance 14 is fixed on a limited area (on the left-side area in Fig. 8(c)) on the surface of the second glass plate 63 which faces the first plate 60 and, on the surface of the first plate 60 facing the second plate 63, two thin gold film electrodes, one as anode 61 for measurement and the other as cathode 62, are installed to directly face the area of the physiologically active substance in the same manner as in Fig. 1 and a comparison electrode 66 in the form of a thin gold film electrode at the part not facing the area of the physiologically active substance. This is an example where electrodes for measurement and for comparison may be installed on a signal plate only with a limitation on their position.

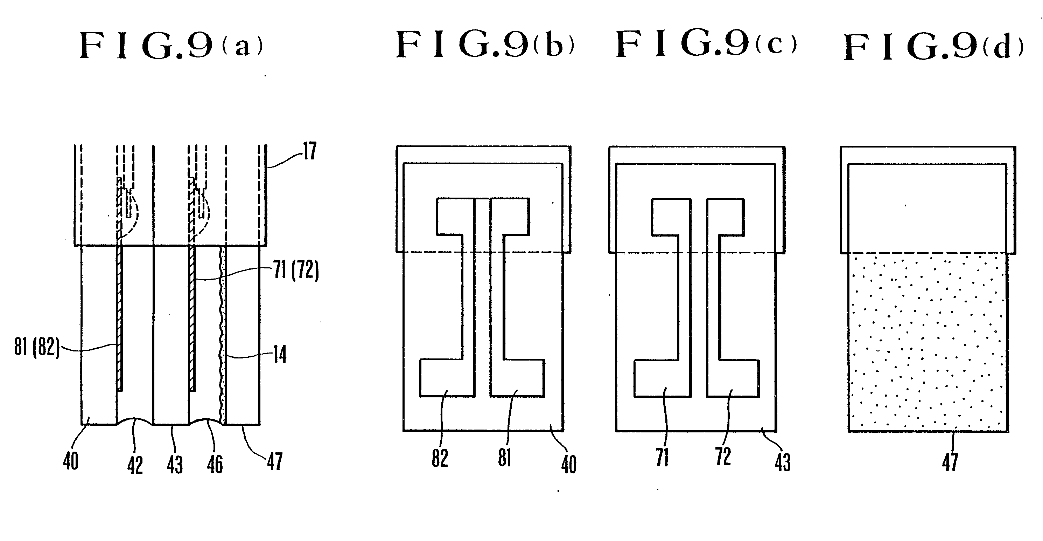

In Figs. 9(a), 9(b), 9(c) and 9(d), an example is illustrated in which a comparison electrode and a room for comparison solution are added to the apparatus in Example 1. It is so composed that an anode 71 and a cathode 72 as measuring electrodes are provided on the first glass plate and an anode 81 and a cathode 82 as comparison electrodes on the third plate.

According to the biosensor of the present invention, a physiologically active substance can be fixed in uniform and stable films on a plate irrespective of the shape of transducer. This invention can also provide a miniaturized biosensor to be used for a minute volume of a sample solution such as, for example, for as minute a sample solution as 3 $\mu$1 which is not in the range of previous apparatus.

If a pair of plates, on one of which a physiologically active substance is fixed and on the other of which a transducer is installed, is combined in plurality , for instance, to form a laminated structure, the resulting biosensor permits a number of items of estimation to be performed at a time with respect to a sample and the biosensor itself can be made smaller in size.

Furthermore, agitation of a sample solution is not required for the estimation. Since the sensing part of transducer makes direct contact with that part of the solution which the concentration of the substance to be measured changes during the progress of reaction, the fluctuation on the precision of estimations is cancelled in spite of the swaying movement and the penetration through membrane of the solution. Thus, accurate estimations can be obtained with high sensitivity of detection.

## Claims

1. A biosensor comprising at least a pair of flat plates placed opposingly to each other forming fine spacing therebetween, a physiologically active substance fixed on a surface of one of the plates facing said space, and a first transducer responsive to a chemical substance provided on a surface of the other plate facing said space, wherein said space is utilized for accommodating a sample solution.

2. A biosensor according to Claim 1 further comprising a second transducer for comparison installed on a surface of one of the plates not facing said space, and a third plate placed opposingly to the surface on which said second transducer for comparison is installed to form therebetween a space for accommodating a sample solution.

3. A biosensor according to Claim 1 or 2, wherein said physiologically active substance is fixed on a limited portion of the surface of said one of the plates, and said first transducer is placed opposing to said limited area where said physiologically active substance is fixed, and further comprising a second transducer for comparison responsive to a chemical substance installed on the surface of the other plate so as to oppose to a remaining portion of the surface of said one of the plate where said physiologically active substance is not fixed.

FIG.1(c)

FIG.1(b)

FIG.1(a)

# F I G. 2

# F I G. 3

FIG.4(a)

E ← → D ← → C

44 (45)

48 (49)

40    41    42    43    46    47

E ← → D ← → C

FIG.4(b)   FIG.4(c)   FIG.4(d)

48    49

47

44    45

43

41

40

FIG.5

50 DRAIN

51 SOURCE

52

# FIG.6

# FIG.7

FIG.8(a)

FIG.8(b)

FIG.8(c)

# FIG.9(a)   FIG.9(b)   FIG.9(c)   FIG.9(d)

EP 0 304 947 A2